# EUROPEAN PATENT APPLICATION

(11) **EP 4 563 195 A1**
(43) Date of publication of application: **04.06.2025**
(21) Application number: 23850365.0
(22) Date of filing: 31.07.2023
(51) Int. Cl.: A61N 5/10

(54) **CAPSULE-TYPE LOW-DOSE RADIATION TREATMENT DEVICE HAVING WHEELCHAIR INTERWORKING STRUCTURE**

(30) Priority: 01.08.2022 KR 20220095621; 13.07.2023 KR 20230091277
(71) Applicant: ReadyCure Inc., Seoul 02792 (KR)
(72) Inventor: CHUNG, Weon Kuu, Seoul 06288 (KR)
(74) Representative: Prüfer & Partner mbB Patentanwälte · Rechtsanwälte
(86) International application number: PCT/KR2023/011108
(87) International publication number: WO 2024/029863

(57) **Abstract**

According to an aspect of the present invention, provided is a capsule-type low-dose radiation therapy device having a wheelchair-linkable structure, wherein a capsule door configured to form the front surface of a radiation therapeutic capsule for providing a radiation-shielded cocoon-shaped therapeutic chamber is fully opened or closed forwards, and a therapeutic bed installation part where a radiation gantry is mounted is coupled to the rear surface of the capsule door to interwork with the capsule door, wherein, when the capsule door is fully opened, a patient-boarding gate is secured by using a lateral space between the therapeutic capsule and the capsule door, and a patient in a wheelchair enters the location of the therapeutic bed installation part through the patient-boarding gate so that the patient is directly transferred from the wheelchair to the therapeutic bed installation part.

## Description

### [Technical Field]

The present invention relates to a radiation therapy device, and more particularly a capsule-type low-dose radiation therapy device having a wheelchair-linkable structure which allows patients with dementia, the elderly, the disabled who have difficulty moving, and the like to easily access a wheelchair and feel emotionally stable.

### [Background Art]

Among radiations generally used for medical purposes, therapeutic radiation is applied to the tumors of cancer patients to prevent cancer cells from reproducing any further, thereby causing cancer cells to die when they reach the end of their lifespan or to alleviate the patient's pain.

Recently, intensity-modulated radiation therapy (IMRT) is gaining attention as a method that modulates the intensity of radiation to suit the shape, size, and location of tumors using a multileaf collimator (MLC) to irradiate radiation prescribed to have optimal energy, thereby reducing side effects of radiation on normal tissue and maximizing treatment outcomes.

However, since these treatment devices used in radiation therapy are manufactured to fit the usage environment of ordinary patients, there was a problem that dementia patients, the elderly, and the disabled had difficulty accessing them using wheelchairs.

In addition, most radiation therapy devices have a sealed structure for radiation shielding, so there was a problem of treatment resistance or psychological anxiety symptoms such as claustrophobia occurring during treatment.

### [Disclosure]

### [Technical Problem]

Therefore, the present invention has been made in view of the above problems, and it is one object of the present invention to provide a capsule-type low-dose radiation therapy device having a wheelchair-linkable structure which allows patients with impaired mobility, such as dementia patients, the elderly, and the disabled, to easily access a radiation therapy device of the present invention using a wheelchair and feel emotionally stable.

It is another object of the present invention to allow a patient to concentrate on radiation therapy with a comfortable mind without feeling claustrophobia or emotional anxiety by manufacturing the entire capsule door with a glass that can shield radiation to provide a sense of openness.

It is yet another object of the present invention to allow a patient to concentrate on radiation therapy with ease without feeling claustrophobia or emotional anxiety, allows the patient to gain psychological stability and, at the same time, to immediately appeal any inconveniences arising during the treatment process to a medical staff outside the therapeutic capsule by allowing direct or indirect communication between the patient and the medical staff through eye contact or body language, and allow the medical staff to observe the patient's condition in real-time, allowing quick response and medical treatment.

### [Technical Solution]

In accordance with an aspect of the present invention, the above and other objects can be accomplished by the provision of A capsule-type low-dose radiation therapy device having a wheelchair-linkable structure, wherein a capsule door that forms a front surface of a radiation therapeutic capsule configured to provide a radiation-shielded cocoon-shaped therapeutic chamber is fully opened or closed forwards, and a therapeutic bed installation part where a radiation gantry is mounted is coupled to a rear surface of the capsule door to interwork with the capsule door, and
when the capsule door is fully opened, a patient-boarding gate is secured due to a lateral space between the therapeutic capsule and the capsule door, and a patient in a wheelchair enters a location of the therapeutic bed installation part through the patient-boarding gate and is directly transferred to the therapeutic bed installation part while riding the wheelchair.

Here, the wheelchair may be provided with a dedicated therapeutic bed in the form of a chair for patient transfer, the therapeutic bed may be transformed into a bed shape and transferred to the therapeutic bed installation part in a state where the patient sits on the therapeutic bed, the therapeutic bed may be unfolded straight and fixed to the therapeutic bed installation part so that the patient receives radiation therapy in a lying position, and after the treatment, the therapeutic bed may be folded into a chair shape and the patient may be loaded onto the wheelchair while sitting.

In accordance with another aspect of the present invention, provided is a capsule-type low-dose radiation therapy device having a wheelchair-linkable structure, including: a radiation therapeutic capsule configured to provide a radiation-shielded cocoon-shaped therapeutic chamber;
a capsule door operated such that a front surface of the therapeutic capsule is fully opened and closed forwards;
a therapeutic bed installation part coupled to an inside of the capsule door, and pulled out together with the capsule door from the therapeutic capsule when the capsule door is opened, so that a patient can board together with the therapeutic bed in the therapeutic capsule while lying down;
a slide driver configured to open or close the therapeutic bed installation part and the capsule door; and
a radiation gantry provided on an upper part of the therapeutic bed installation part to surround a patient's body in a circular shape so that the patient's body can be irradiated with low-dose radiation.

In addition, the therapeutic capsule may form a radiation-shielded therapeutic chamber in a silkworm cocoon shape extended in a longitudinal direction so that a patient can receive radiation therapy while lying down, and an entrance may be formed to fully open a front surface of the therapeutic chamber in a short axis direction of the therapeutic chamber so that a patient can enter and exit the entrance, and lighting may be formed on a ceiling of the therapeutic chamber.

Here, the entire capsule door may be made of lead-containing glass capable of radiation shielding.

In addition, the radiation gantry may form a ring-shaped gate to surround a patient's body so that therapeutic radiation is irradiated toward the patient's body from an inner periphery direction of the gate, and 10 to 40 nanotube sources for irradiating radiation may be arranged in a circular manner around the inner periphery of the gate, and the respective nanotube sources may operate sequentially in a circumferential direction in a manner of irradiating radiation for about 1 second.

Here, the nanotube sources may be arranged in multiple rows around the inner periphery of the gate of the radiation gantry, and each nanotube source array may operate alternately.

### [Advantageous effects]

The present invention provides a patient-friendly treatment environment that allows patients with impaired mobility, such as dementia patients, the elderly, and the disabled, to easily access a radiation therapy device of the present invention using a wheelchair and feel emotionally stable, thereby meeting customer needs.

In addition, the present invention allows a patient to concentrate on radiation therapy with a comfortable mind without feeling claustrophobia or emotional anxiety by manufacturing the entire capsule door with a glass that can shield radiation to provide a sense of openness.

Further, the present invention allows a patient to concentrate on radiation therapy with ease without feeling claustrophobia or emotional anxiety, allows the patient to gain psychological stability and, at the same time, to immediately appeal any inconveniences arising during the treatment process to a medical staff outside the therapeutic capsule by allowing direct or indirect communication between the patient and the medical staff through eye contact or body language, and allows the medical staff to observe the patient's condition in real-time, allowing quick response and medical treatment.

### [Description of Drawings]

FIG. 1 illustrates the perspective view of a capsule-type low-dose radiation therapy device having a wheelchair-linkable structure according to the present invention in a state where its capsule door is closed.
FIG. 2 illustrates the perspective view of the capsule-type low-dose radiation therapy device having a wheelchair-linkable structure according to the present invention in a state where its capsule door is open.
FIG. 3 illustrates the perspective view of a patient in a wheelchair according to the present invention who enters the capsule-type low-dose radiation therapy device.
FIG. 4 illustrates the perspective view of a wheelchair-coupled therapeutic bed according to the present invention in a state of being unfolded into a bed structure.
FIG. 5 illustrates the perspective view of a state in which the wheelchair-coupled therapeutic bed according to the present invention is loaded together with a patient into a therapeutic capsule.
FIG. 6 illustrates the perspective view of a state in which the therapeutic bed according to the present invention is loaded together with a patient into the therapeutic capsule.
FIG. 7 illustrates the perspective view of a state in which a medical staff operates a controller while the therapeutic bed according to the present invention is loaded together with a patient into the therapeutic capsule.
FIG. 8 is a conceptual diagram illustrating the arrangement of nanotube sources of the low-dose radiation therapy device according to the present invention

### [Best Mode]

Hereinafter, the present invention according to a preferred embodiment will be described in detail with reference to the attached drawings. Here, the same symbols are used for the same components, and repetitive descriptions and detailed descriptions of known functions and configurations that may unnecessarily obscure the gist of the invention are omitted. Embodiments of the invention are provided to more completely explain the present invention to those with average knowledge in the art. Therefore, the shapes and sizes of elements in the drawings may be exaggerated for clearer explanation.

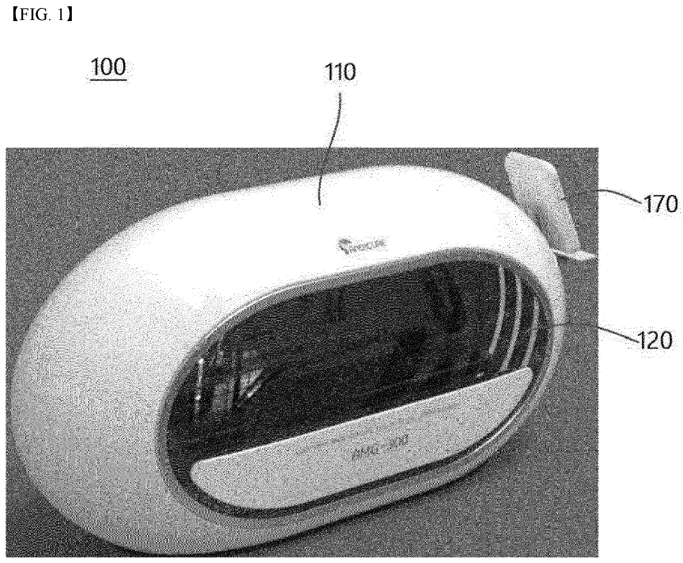

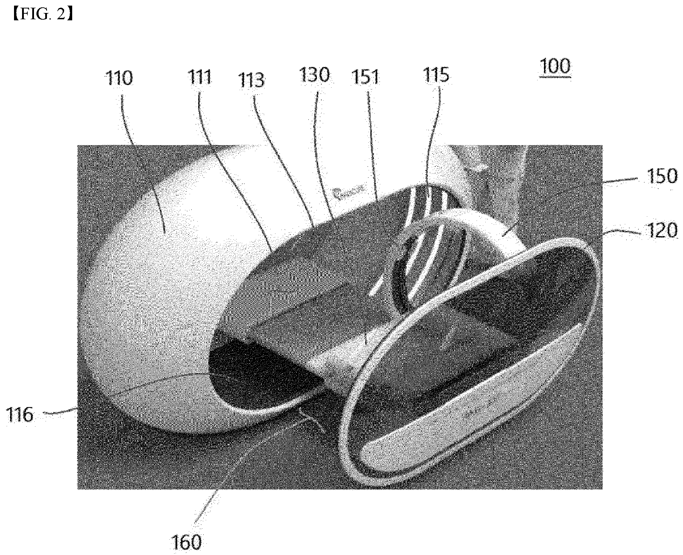

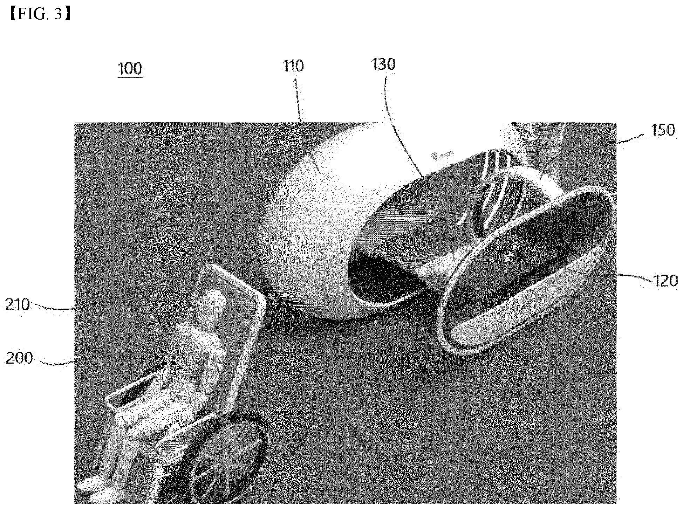

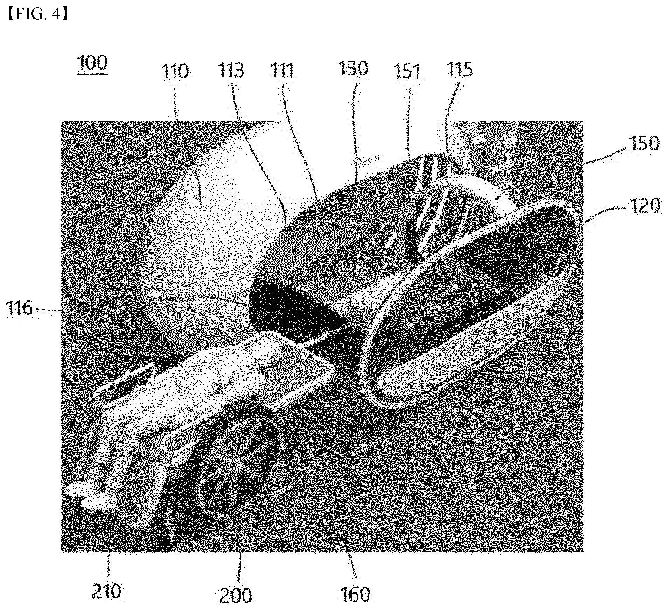

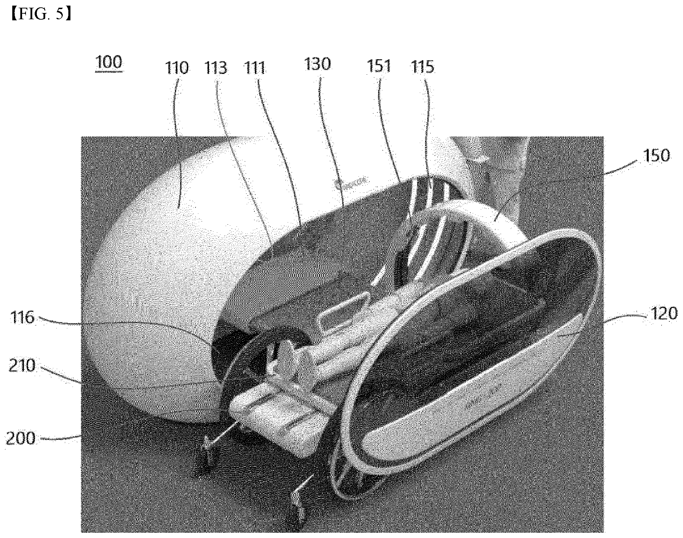

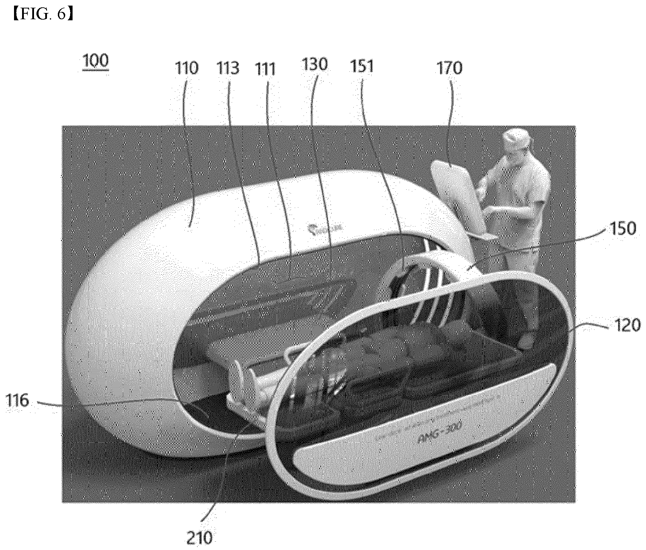

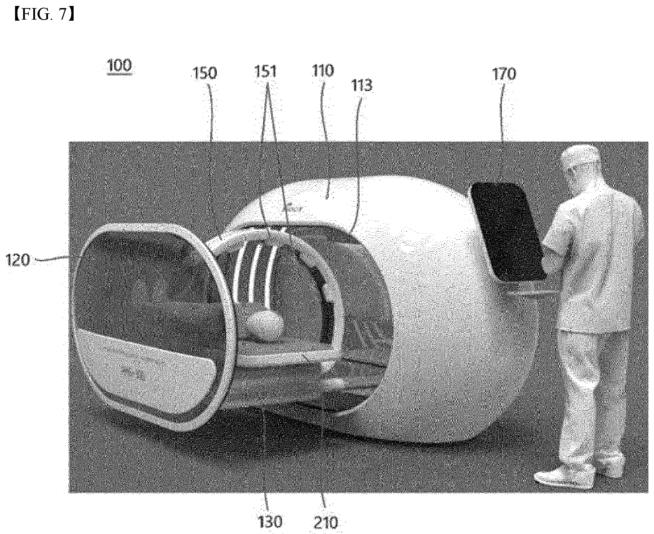

FIG. 1 illustrates the perspective view of a capsule-type low-dose radiation therapy device having a wheelchair-linkable structure according to the present invention in a state where its capsule door is closed, FIG. 2 illustrates the perspective view of the capsule-type low-dose radiation therapy device having a wheelchair-linkable structure according to the present invention in a state where its capsule door is open, FIG. 3 illustrates the perspective view of a patient in a wheelchair according to the present invention who enters the capsule-type low-dose radiation therapy device, FIG. 4 illustrates the perspective view of a wheelchair-coupled therapeutic bed according to the present invention in a state of being unfolded into a bed structure, FIG. 5 illustrates the perspective view of a state in which the wheelchair-coupled therapeutic bed according to the present invention is loaded together with a patient into a therapeutic capsule, FIG. 6 illustrates the perspective view of a state in which the therapeutic bed according to the present invention is loaded together with a patient into the therapeutic capsule, and FIG. 7 illustrates the perspective view of a state in which a medical staff operates a controller while the therapeutic bed according to the present invention is loaded together with a patient into the therapeutic capsule.

Referring to FIGS. 1 to 7, a capsule-type low-dose radiation therapy device 100 having a wheelchair-linkable structure according to the present invention may be largely composed of a therapeutic capsule 110, a capsule door 120, a therapeutic bed installation part 130, a radiation gantry 150, a wheelchair 200, and a therapeutic bed 210.

First, the appearance of the radiation therapeutic capsule 110 may have a silkworm cocoon-shaped and dome-shaped structure. Such a silkworm cocoon-shaped and dome-shaped structure can provide psychological comfort and ease to patients due to its streamlined appearance.

In addition, the silkworm cocoon-shaped and dome-shaped structure has the advantage of allowing a stable structural design. For example, such a structure is suitable for mass production because the integrated design and molding of inner and outer cases are possible, and the assembly process may be simplified by minimizing the connections between case materials, which provides the advantage of reducing production costs by minimizing production processes and production facilities.

In addition, a structure with excellent sealing may be provided by minimizing the connections between case materials, which provides an advantageous advantage in the design of the self-radiation shielding structure of the present invention that can prevent radiation from leaking during a radiation treatment process.

The therapeutic capsule 110 forms the therapeutic chamber 111 such that a patient can lie down inside and receive radiation therapy. Here, the therapeutic chamber 111 provides a radiation shielding space in the shape of a silkworm cocoon similar to the appearance of the therapeutic capsule 110, and its ceiling height can be formed as low as possible so that a patient can feel as comfortable as possible when lying down, and can be formed to have the height of the patient's sitting height.

In other words, the therapeutic capsule 110 forms the radiation-shielded therapeutic chamber 111 in the shape of a silkworm cocoon that is extended in a longitudinal direction so that the patient can receive radiation therapy while lying down. The patient may enter and exit the therapeutic chamber 111 while lying down. For this, the front surface of the therapeutic chamber 111 in a short axis direction may be fully opened to form an entrance 113 through which the patient can enter and exit.

Here, the entrance 113 may be formed in an oval or track shape extended in the longitudinal direction.

Here, the internal light 115 may be formed on the ceiling of the therapeutic chamber 111, and the light 115 may form a track-shaped lighting line along the inner surface from the ceiling to the floor.

In addition, the floor of the therapeutic chamber 111 may be provided with an auxiliary room 116 space where various components for operating the radiation therapy device are accommodated.

In addition, the therapeutic bed installation part 130 may be formed on the rear inner wall of the therapeutic chamber 111 such that the therapeutic bed 210 can be boarded together with a patient. Here, the therapeutic bed installation part 130 may provide a slide structure that can be expanded in the short axis direction of the therapeutic capsule 110, and the therapeutic bed 210 having a bed structure may be detachably mounted on the therapeutic bed installation part 130 such that a patient can lie down and receive radiation therapy, and may be slid together with the therapeutic bed installation part 130.

Here, the height of the therapeutic bed installation part 130 may be formed as the chair height of the wheelchair 200. When the therapeutic bed installation part 130 is formed to have such a height, it is possible to provide convenience so that a patient in the wheelchair 200 can more easily be moved to and boarded on the therapeutic bed installation part 130.

In addition, the capsule door 120 may be installed such that the front surface of the therapeutic capsule 110 is fully opened and closed.

Here, the capsule door 120 may combined with the therapeutic bed installation part 130 and may be operated to open or close forwards.

Here, the entire capsule door 120 may be made of a lead-containing glass capable of radiation shielding, may be manufactured in the same shape as the entrance 113 of the therapeutic capsule 110 to open and close the entrance 113, and may be formed in an oval or track shape extended in the longitudinal direction as shown in the accompanying drawings.

By forming the entire capsule door 120 with glass capable of shielding radiation to provide a sense of openness, a patient can concentrate on radiation therapy with ease without feeling claustrophobia or emotional anxiety, and the patient can gain psychological stability and, at the same time, can immediately appeal any inconveniences arising during the treatment process to a medical staff outside the therapeutic capsule by allowing direct or indirect communication between the patient and the medical staff through eye contact or body language, and the medical staff can observe the patient's condition in real-time, allowing quick response and medical treatment.

One end of the slide of the therapeutic bed installation part 130 is coupled to the inside of the capsule door 120, so that, when the capsule door 120 is opened, the therapeutic bed installation part 130 is pulled out together to the outside of the therapeutic capsule 110, so that the patient can board the treatment bed while lying down.

Here, the therapeutic bed installation part 130 may be expanded by manual operation, or may be automatically slide by a medical staff's button operation.

Here, a slide driver may be further included to automatically open or close the therapeutic bed installation part 130 and the capsule door 120 by button operation.

Here, the slide driver may be configured to use a piston expansion mechanism using a hydraulic device installed in the therapeutic bed installation part 130 or to employ the linear motion structure of a rack and pinion gear using the rotational power of a motor.

Of course, the slide driver may have various structures that flexibly operate, in addition to the above example form.

In addition, the therapeutic bed installation part 130 may be installed with the radiation gantry 150 that can irradiate low-dose radiation. Here, FIGS. 1 to 7 illustrates the radiation gantry 150 installed at the patient's head position as an example. This type of installation may be suitable for cases where radiation therapy is intensively performed on the patient's head, such as in the case of dementia patients. However, the installation location of the radiation gantry 150 is not limited to this example, and may vary depending on the patient's treatment site.

Here, the radiation gantry 150 may be provided in a ring-shaped structure to wrap around the patient's body site to be treated and to irradiate the site with the therapeutic radiation.

The radiation gantry 150 forms a ring-shaped gate surrounding the patient's body so that therapeutic radiation is irradiated toward the patient's body from the inner periphery of the gate, and 10 to 40 carbon nanotube sources 151 for irradiating radiation may be arranged in a circular shape around the inner periphery of the gate.

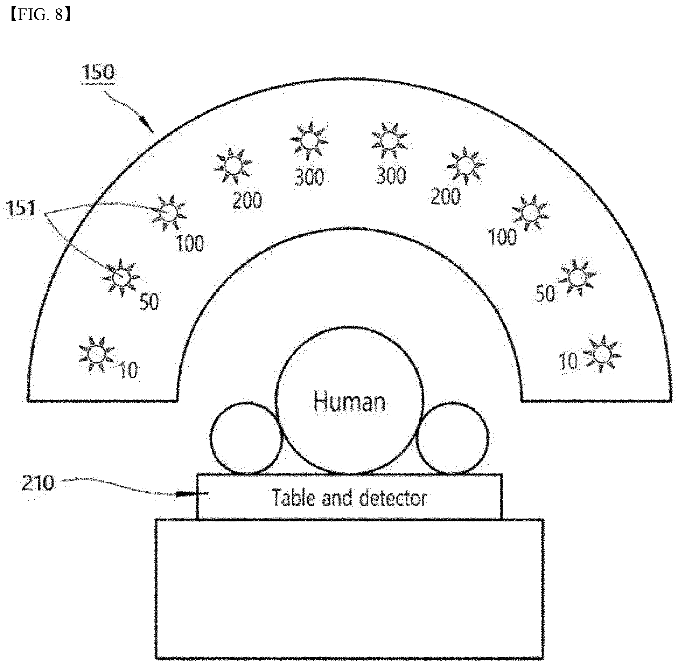

FIG. 8 is a conceptual diagram illustrating the arrangement of nanotube sources of the low-dose radiation therapy device according to the present invention.

Referring to FIG. 8, a plurality of nanotube sources 151 emitting low energy of 10 to 300 KVP (kilovolt peak) and low-dose radiation of 10 to 100 Mu/min may be arranged by band around the inner periphery of the radiation gantry 150.

Here, low-dose radiation (LDR) refers to radiation having low intensity and is close to nature. Large doses of radiation can be harmful to living organisms, but small doses of LDR can actually promote the physiological activities of living organisms, extending lifespan, promoting growth, or reducing tumor incidence. This is called radiation hormesis.

It was reported that LDR in the range of miligray (mGy) induces resistance to disease and exhibits adaptive protection effects, unlike high-dose radiation (Sakai K., et al., Dose Response, 2006, 4(4):327~332; Sakai K., et al, Int. J. Low Radiat., 2003, 1(1):142~146; Scott BR., Dose Response, 2008 6(3):299~318), and there is a report that when a certain amount of LDR was administered to experimental mice with diabetes genes, the disease was improved (Sakai K., et al., Dose Response, 2006, 4(4):327~332). In addition, radiation of 0.25 Gy or less has the effect of protecting DNA damage and repairing damaged DNA (Le XC., et al., Science, 1998, 280(5366):1066~1069), and LDR is known to have an immune-enhancing effect (Nogami M., et al., Int. J. Radiat. Biol., 1993, 63(6):775~783; Nogami M., et al., Radiat. Res., 1994, 139(1):47~52).

Referring to FIG. 8, the nanotube sources 151 may be arranged such that the highest energy is emitted from the center of the radiation gantry 150, and gradually lower energy is emitted as approaching ends on both sides from the center of the radiation gantry 150.

For example, in the case where 10 nanotube sources 151 are arranged radially as in FIG. 8, a pair of nanotube sources 151 installed at two symmetrical points in the center of the radiation gantry 150 may be arranged to respectively emit 300 KVP of energy, a left and right symmetrical pair of nanotube sources located under the points may be arranged to respectively emit 200 KVP of energy, a left and right symmetrical pair of nanotube sources located under the points may be arranged to respectively emit 100 KVP of energy, a left and right symmetrical pair of nanotube sources located under the points may be arranged to respectively emit 50 KVP of energy, and a left and right symmetrical pair of nanotube sources located under the points may be arranged to respectively emit 10 KVP of energy.

Meanwhile, the present invention may include a controller 170 for selectively controlling the nanotube sources 151. Here, the controller 170 may be a portable terminal capable of network use.

Through such a controller 170, a selective treatment mode may be set according to the type of disease and the patient's condition.

For example, there is a need to use different intensity and irradiation band of radiation for each treatment, such as skin cancer treatment, benign disease-keloid, arthritis, heterotopic ossification, psoriasis, atopy, dementia, Parkinson's disease, and animal cancer treatment. For this, among the plural nanotube sources 151 divided by band, a nanotube source 151 of a specific band may be selectively operated, or two or more nanotube sources 151 may be operated simultaneously to achieve combined treatment.

Considering the patient's age or health condition, various treatment modes may be set in advance and operated, and the selective operation of the plural nanotube sources according to the set treatment mode and the total amount and number of times of radiation to be irradiated may be comprehensively controlled through the operation of the controller 170.

The controller 170 may be installed on the longitudinal outer surface of the therapeutic capsule 110 as shown in FIGS. 1 to 7, so that an operator can conveniently operate and use it.

As described above, the nanotube sources 151 may be a carbon nanotube X-ray source including an emitter made of a metal and carbon nanotubes (CNTs) grown from the emitter.

Meanwhile, in the present invention, the respective nanotube sources 151 may be sequentially operated in a circumferential direction in a manner of irradiating radiation for about 1 second.

Here, the beam energy and dose irradiated from the nanotube sources 151 may be irradiated with the same energy and dose.

In addition, the nanotube sources 151 may be arranged in multiple rows around the inner periphery of the gate of the radiation gantry 150.

Here, the nanotube sources 151 may be arranged to operate alternately.

For example, the radiation therapy may be performed sequentially in the manner that, after the radiation irradiation of the nanotube sources 151 of the first row is sequentially performed, the nanotube sources 151 of the first row are in a standby state, and the radiation irradiation of the nanotube sources 151 of the second row in a standby state is performed.

When radiation therapy is performed alternately using the nanotube sources 151 of the first and second rows in this way, the treatment time may be shortened and intensive radiation therapy may be achieved.

Hereinafter, the operation of the present invention is described as follows.

First, as shown in FIG. 2, the capsule door 120 forming the front surface of the radiation therapeutic capsule 110 is fully opened forward by the operation of the controller 170 by a medical staff or a manual operation.

Here, the therapeutic bed installation part 130 equipped with the radiation gantry 150 is connected to the rear surface of the capsule door 120 and is interworked with the capsule door 120 to slide forwards together with the capsule door 120.

Here, when the capsule door 120 is fully opened, a lateral space between the therapeutic capsule 110 and the capsule door 120 is used to secure the space of a patient-boarding gate 160.

Next, as shown in FIG. 3, a patient enters the therapeutic bed installation part 130 while riding the wheelchair 200 through the patient-boarding gate 160. Here, the patient can move by operating the wheelchair 200 by himself or with the help of medical staff.

Here, the wheelchair 200 is provided with a dedicated therapeutic bed 210 for patient transport which can be folded into a chair shape or unfolded into a bed shape.

Next, referring to FIGS. 4 to 7, when the therapeutic bed 210 is unfolded and fixed, the therapeutic bed 210 may be transformed into a bed shape and transferred to the therapeutic bed installation part 130 in a state where the patient is sitting on the therapeutic bed 210, and the therapeutic bed 210 may be unfolded straight and fixed to the therapeutic bed installation part 130, so that the patient can receive radiation therapy while lying down.

After the radiation therapy, the therapeutic bed 210 may be folded into a chair shape so that the patient can be loaded onto the wheelchair 200 while sitting.

As discussed above, the present invention provides a patient-friendly treatment environment that allows patients with impaired mobility, such as dementia patients, the elderly, and the disabled, to easily access a radiation therapy device of the present invention using a wheelchair and feel emotionally stable, thereby meeting customer needs. In addition, the present invention allows a patient to concentrate on radiation therapy with a comfortable mind without feeling claustrophobia or emotional anxiety by manufacturing the entire capsule door with a glass that can shield radiation to provide a sense of openness. Further, the present invention allows a patient to concentrate on radiation therapy with ease without feeling claustrophobia or emotional anxiety, allows the patient to gain psychological stability and, at the same time, to immediately appeal any inconveniences arising during the treatment process to a medical staff outside the therapeutic capsule by allowing direct or indirect communication between the patient and the medical staff through eye contact or body language, and allows the medical staff to observe the patient's condition in real-time, allowing quick response and medical treatment.

The present invention has been described with reference to an embodiment shown in the attached drawings, but this is merely illustrative, and those skilled in the art will recognize that various modifications and other equivalent embodiments are possible therefrom. Therefore, the true scope of protection of the present invention should be determined only by the appended claims.

## Claims

1. A capsule-type low-dose radiation therapy device having a wheelchair-linkable structure, wherein a capsule door that forms a front surface of a radiation therapeutic capsule configured to provide a radiation-shielded cocoon-shaped therapeutic chamber is fully opened or closed forwards, and a therapeutic bed installation part where a radiation gantry is mounted is coupled to a rear surface of the capsule door to interwork with the capsule door, and
when the capsule door is fully opened, a patient-boarding gate is secured due to a lateral space between the therapeutic capsule and the capsule door, and a patient in a wheelchair enters a location of the therapeutic bed installation part through the patient-boarding gate and is directly transferred to the therapeutic bed installation part while riding the wheelchair.

2. The capsule-type low-dose radiation therapy device according to claim 1, wherein the wheelchair is provided with a dedicated therapeutic bed in the form of a chair for patient transfer, the therapeutic bed is transformed into a bed shape and transferred to the therapeutic bed installation part in a state where the patient sits on the therapeutic bed, the therapeutic bed is unfolded straight and fixed to the therapeutic bed installation part so that the patient receives radiation therapy in a lying position, and after the treatment, the therapeutic bed is folded into a chair shape and the patient is loaded onto the wheelchair while sitting.

3. A capsule-type low-dose radiation therapy device having a wheelchair-linkable structure, comprising:
a radiation therapeutic capsule configured to provide a radiation-shielded cocoon-shaped therapeutic chamber;
a capsule door operated such that a front surface of the therapeutic capsule is fully opened and closed forwards;
a therapeutic bed installation part coupled to an inside of the capsule door, and pulled out together with the capsule door from the therapeutic capsule when the capsule door is opened, so that a patient can board together with the therapeutic bed in the therapeutic capsule while lying down;
a slide driver configured to open or close the therapeutic bed installation part and the capsule door; and
a radiation gantry provided on an upper part of the therapeutic bed installation part to surround a patient's body in a circular shape so that the patient's body can be irradiated with low-dose radiation.

4. The capsule-type low-dose radiation therapy device according to claim 1 or claim 3, wherein the therapeutic capsule forms a radiation-shielded therapeutic chamber in a silkworm cocoon shape extended in a longitudinal direction so that a patient can receive radiation therapy while lying down, and an entrance is formed to fully open a front surface of the therapeutic chamber in a short axis direction of the therapeutic chamber so that a patient can enter and exit the entrance, and lighting is formed on a ceiling of the therapeutic chamber.

5. The capsule-type low-dose radiation therapy device according to claim 4, wherein the entire capsule door is made of lead-containing glass capable of radiation shielding.

6. The capsule-type low-dose radiation therapy device according to claim 1 or claim 3, wherein the radiation gantry forms a ring-shaped gate to surround a patient's body so that therapeutic radiation is irradiated toward the patient's body from an inner periphery direction of the gate, and 10 to 40 nanotube sources for irradiating radiation are arranged in a circular manner around the inner periphery of the gate, and the respective nanotube sources operate sequentially in a circumferential direction in a manner of irradiating radiation for about 1 second.

7. The capsule-type low-dose radiation therapy device according to claim 6, wherein the nanotube sources are arranged in multiple rows around the inner periphery of the gate of the radiation gantry, and each nanotube source array operates alternately.
